# EUROPEAN PATENT APPLICATION

(11) **EP 1 621 209 A2**
(43) Date of publication of application: **01.02.2006**
(21) Application number: 05017841.7
(22) Date of filing: 21.10.1999
(51) Int. Cl.: A61K 39/395, C07K 16/42

(54) **Vaccines based on domains of chimeric immunoglobulin E peptides**

(30) Priority: 02.11.1998 US 106652 P; 22.09.1999 US 401636
(62) Divisional of application: 99963705.1
(71) Applicant: Resistentia Pharmaceuticals AB, 751 08 Uppsala (SE)
(72) Inventor: Hellman, Lars T., 753 29 Uppsala (SE)
(74) Representative: Cornish, Kristina Victoria Joy

(57) **Abstract**

The invention relates to methods and materials involved in the treatment and prevention of various diseases such as infections and IgE-related diseases. Specifically, the invention relates to methods and materials that can be used to vaccinate a mammal against specific self or non-self antigens. For example, the methods and materials described herein can be used to reduce the effects of IgE antibodies within a mammal by reducing the amount of total and receptor bound IgE antibodies in the mammal. In addition, the invention provides vaccine conjugates, immunogenic polypeptides, nucleic acid molecules that encode immunogenic polypeptides, host cells containing the nucleic acid molecules, that encode immunogenic polypeptides, and methods for making vaccine conjugates and immunogenic polypeptides as well as nucleic acid molecules that encode immunogenic polypeptides based on the use of IgE domains from non-placental mammals. Further, the invention provides an IgE vaccine that induces an anti-self IgE response in a mammal.

## Description

### BACKGROUND

### I. Technical Field

The invention relates to methods and materials involved in the treatment of various diseases such as infections and IgE-related diseases. Specifically, the invention relates to methods and materials that can be used to vaccinate a mammal against specific self or non-self antigens. For example, the methods and materials described herein can be used to reduce the effects of IgE antibodies within a mammal.

### 2. Background Information

Mammals are susceptible to many diseases and illnesses including bacterial infections, viral infections, and IgE-related diseases such as allergies. In general, infections are characterized by the invasion and multiplication of microorganisms (e.g., bacteria, fungi, and viruses) within body tissues. Many types of infections can be treated or prevented by the use of vaccines. For example, the polio vaccine can prevent poliovirus infections. Typically, a vaccine is a suspension of attenuated or killed microorganisms.

IgE-related diseases are mediated by a class of immunoglobulin designated as immunoglobulin E (IgE). In fact, IgE antibodies are a major cause of hypersensitivity reactions found within the human population despite their normally very low concentration in human plasma (10-400 ng/mL). The effects are due to the interaction of IgE antibodies with the high-affinity receptor for IgE on mast cells and basophilic leukocytes. Cross-linking of two IgE receptors on the surface of these cell types, for example by allergen binding, initiates the release of a number of physiologically active substances such as histamine, PAF (platelet activating factor), heparin, leukotrienes, prostaglandins, thromboxanes, and chemotactic factors for eosinophilic and neutrophilic granulocytes. Presumably, these mediators cause the direct symptoms of IgE-mediated allergic reactions (type I hypersensitivity). Disease conditions belonging to this group can include asthma, fur allergies, pollen allergies, food allergies, and eczema.

The high-affinity receptor for IgE has been characterized. This receptor appears to be present on mast cells, basophilic leucocytes, eosinophils, monocytes, and Langerhan cells. In addition, the receptor is a complex of three different subunits (α, β, and γ chains). The α chain is localized mainly extra-cellularly and appears to interact with the IgE molecule. Previous studies of the epsilon chain of the IgE molecule have suggested that a region of 76 amino acids at the border between the CH2 and CH3 domains (CH refers to the constant domains in the heavy chain) is important for the interaction between the IgE molecule and its high-affinity receptor. In addition, a peptide corresponding to this region was shown to inhibit the interaction between native IgE and its high-affinity receptor *in vitro* at a molar ratio of nearly 1:1 compared to the whole CH2-CH3-CH4 region (Helm *et al*., *Nature* 331, 180-183 (1988)). This peptide was also shown to inhibit an IgE-mediated flare reaction in allergen stimulation. In this case, however, the concentration was about 10 times the concentration needed to exhibit the same inhibitory effect with native IgE (Helm *et al*., *Proc. Natl. Acad. Sci. USA* 86, 9465-9469 (1989)).

### SUMMARY

The invention relates to methods and materials involved in the treatment and prevention of various diseases such as infections and IgE-related diseases. Specifically, the invention relates to methods and materials that can be used to vaccinate a mammal against specific self or non-self antigens. For example, the methods and materials described herein can be used to reduce the effects of IgE antibodies within a mammal by reducing the amount of total and receptor bound IgE antibodies in the mammal. Such methods and materials can be used to treat atopic allergies in mammals such as humans, dogs, and pigs.

The invention is based on the discovery that a vaccine conjugate can be designed to contain at least two polypeptides with each polypeptide having at least two similar amino acid segments such that the administration of the conjugate to a mammal can induce an immune response against at least a portion of one of the polypeptides. Such immune responses can be more potent than the responses induced by any of the polypeptides in an unconjugated form or any conjugate of polypeptides lacking at least two similar amino acid segments. Thus, the vaccine conjugates described herein can be used to provide mammals with substantial protection against a wide range of either self (e.g., IgE molecules) or non-self (e.g., viral polypeptides) antigens.

The invention also is based on the discovery that a vaccine conjugate can be designed to contain a polypeptide having a cytokine activity such that a potent immune response is induced against another polypeptide within the conjugate. Such immune responses can be more potent than the responses induced by a conjugate lacking a polypeptide having a cytokine activity. Although not limited to any particular mode of action, a conjugate containing a polypeptide having a cytokine activity as well as an immunogenic polypeptide presumably concentrates cytokine activity to the localized area containing the immunogenic polypeptide. Thus, the polypeptide having cytokine activity can stimulate cells that participate in generating a specific immune response against the immunogenic polypeptide.

In addition the invention is based on the discovery that polypeptides containing a self IgE portion and a non-self IgE portion are immunogenic and induce an effective anti-self IgE response in mammals. Such immunogenic polypeptides can be used as a vaccine to induce an anti-self IgE response that counteracts the hypersensitivity induced by self IgE antibodies. Although not limited to any particular mode of action, the immunogenic polypeptides described herein induce the production of anti-self IgE antibodies that presumably have specificity for the portion of the IgE molecule that interacts with the high-affinity IgE receptor. After production, the anti-self IgE antibodies can interact with the self-IgE antibodies such that the self IgE antibodies are unable to bind to the high-affinity IgE receptor. This inhibition of receptor binding presumably reduces the hypersensitivity induced by self IgE antibodies. Thus, the degree of IgE-induced effects can be reduced as more anti-self IgE antibodies are produced.

In general, the invention features an immunogenic polypeptide having a self IgE portion and a non-self IgE portion. The immunogenic polypeptide is effective to induce an anti-self IgE response in a mammal (e.g., human). The self portion can contain at least a portion of a CH3 domain of IgE. The polypeptide can be capable of dimerizing to form a soluble immunogenic dimer effective to induce the anti-self IgE response in the mammal. The non-self IgE portion can contain a first region and a second region with the self IgE portion being located between the first and second regions of the non-self IgE portion. The first region can contain at least a portion of an IgE CH2 domain, and the second region can contain at least a portion of an IgE CH4 domain. The non-self IgE portion can contain an IgE sequence present in a non-placental mammal (e.g., opossum, platypus, koala, kangaroo, wallaby, and wombat). The self IgE portion can lack the CH2 domain of an IgE antibody. The immunogenic polypeptide can contain a eukaryotic post-translational modification. In addition, the immunogenic polypeptide can contain a polyhistidine sequence. The anti-self IgE response can be a polyclonal response.

In another embodiment, the invention features a nucleic acid molecule containing a nucleic acid sequence that encodes an immunogenic polypeptide. The immunogenic polypeptide contains a self IgE portion as well as a non-self IgE portion, and is effective to induce an anti-self IgE response in a mammal. The nucleic acid molecule can contain an additional nucleic acid sequence that encodes an amino acid sequence that promotes the secretion of the immunogenic polypeptide from a eukaryotic cell.

Another embodiment of the invention features a host cell (e.g., eukaryotic cell) containing a nucleic acid molecule that has a nucleic acid sequence that encodes an immunogenic polypeptide. The immunogenic polypeptide contains a self IgE portion as well as a non-self IgE portion, and is effective to induce an anti-self IgE response in a mammal.

Another embodiment of the invention features a soluble immunogenic dimer containing two immunogenic polypeptides that are capable of dimerizing to form the soluble immunogenic dimer. Each of the two immunogenic polypeptides contains a self IgE portion and a non-self IgE portion, and the soluble immunogenic dimer is effective to induce an anti-self IgE response in a mammal.

Another embodiment of the invention features a vaccine containing an immunogenic polypeptide having a self IgE portion and a non-self IgE portion. The immunogenic polypeptide is effective to induce an anti-self IgE response in a mammal. The vaccine can contain a pharmaceutically acceptable carrier.

Another embodiment of the invention features a method for making a nucleic acid molecule that encodes an immunogenic polypeptide effective to induce an anti-self IgE response in a mammal. The method includes combining first and second nucleic acid sequences to form the nucleic acid molecule, where the first nucleic acid sequence encodes at least a portion of an IgE molecule present within the mammal, and where the second nucleic acid sequence encodes at least a portion of an IgE molecule not present in the mammal.

Another embodiment of the invention features a method for making a nucleic acid molecule that encodes an immunogenic polypeptide effective to induce an anti-self IgE response in a mammal. The method includes (a) selecting a first nucleic acid sequence, where the first nucleic acid sequence encodes at least a portion of an IgE molecule present within the mammal, (b) selecting a second nucleic acid sequence, where the second nucleic acid sequence encodes at least a portion of an IgE molecule not present in the mammal, and (c) combining the first and second nucleic acid sequences to form the nucleic acid molecule.

In another aspect, the invention features a vaccine complex for vaccinating a mammal (e.g., human). The complex contains a first and second polypeptide. Each of the first and second polypeptides contains at least two similar amino acid sequences at least five amino acid residues in length. In addition, the first and second polypeptides are connected to form the complex, and administration of the complex to the mammal induces an immune response against at least a portion of the first or second polypeptide. The first and/or second polypeptide can contains an amino acid sequence expressed by the mammal. The first and second polypeptides can be identical, and can form a dimer. The connection of the first and second polypeptides can include a disulfide bond. The connection of the first and second polypeptides can include a non-covalent interaction. The first and/or second polypeptide can contain a linker site (e.g., a polyhistidine sequence). The amino and carboxyl termini of the first and/or second polypeptide can contain the linker site. The complex can include a linking molecule (e.g., an antibody such as an anti-polyhistidine antibody). A linking molecule can connects the first and second polypeptide. The complex can contain a third polypeptide, where the third polypeptide has a cytokine activity. The cytokine activity can be an activity of a cytokine such as interferon-α, interferon-β, interferon-γ, TNF-α, IL-1, IL-2, IL-4. IL-6. IL-12, IL-15, IL-18, and granulocyte-macrophage colony stimulating factor. A linking molecule can connect the third polypeptide to the first or second polypeptide. The similar amino acid sequences can be greater than about twenty amino acid residues in length. The complex can contain an Fc-gamma receptor II blocking molecule (e.g., an anti-CD32 antibody).

In another embodiment the invention features a vaccine complex for vaccinating a mammal (e.g., human). The complex contains a first polypeptide connected to a second polypeptide, where the first polypeptide contains at least two similar amino acid sequences at least five amino acids in length. In addition, the second polypeptide has a cytokine activity, and administration of the complex to the mammal induces an immune response against at least a portion of the first polypeptide. The first polypeptide can contain an amino acid sequence expressed by the mammal. The connection of the first and second polypeptides can include a non-covalent interaction. The first and/or second polypeptide can contain a linker site (e.g., a polyhistidine sequence). For example, the amino and carboxyl termini of the first polypeptide can contain a linker site. The complex can contain a linking molecule (e.g., an antibody such as an anti-polyhistidine antibody). The cytokine activity can be an activity of a cytokine such as interferon-α, interferon-β, interferon-y, TNF-α, IL-1, IL-2, IL-4, IL-6, IL-12, IL-15, IL-18, and granulocyte-macrophage colony stimulating factor. The complex can contain a third polypeptide. The first and third polypeptides can be identical and can form a dimer. The connection of the first and third polypeptides can include a disulfide bond. The similar amino acid sequences can be greater than about twenty amino acid residues in length. The complex can contain an Fc-gamma receptor II blocking molecule (e.g., an anti-CD32 antibody).

Another embodiment of the invention features a vaccine complex for vaccinating a mammal (e.g., human). The complex contains a first, second, and third polypeptide, where the first, second, and third polypeptides are connected to form the complex. The first polypeptide has a first cytokine activity. The second polypeptide has a second cytokine activity. The administration of the complex to the mammal induces an immune response against at least a portion of the third polypeptide. The third polypeptide can contain an amino acid sequence expressed by the mammal. The connections of the first, second, and third polypeptides can include non-covalent interactions. The first, second, and/or third polypeptide can contain a linker site. The complex can contain a linking molecule. The third polypeptide can contain at least two similar amino acid sequences at least five amino acids in length. The complex can contain an Fc-gamma receptor II blocking molecule (e.g., an anti-CD32 antibody).

Another embodiment of the invention features a vaccine complex for vaccinating a mammal (e.g., human). The complex contains a first polypeptide connected to a second polypeptide, where the first polypeptide is a polypeptide having interferon-α or interferon-β activity, and administration of the complex to the mammal induces an immune response against at least a portion of the second polypeptide. The second polypeptide can contain an amino acid sequence expressed by the mammal. The connection of the first and second polypeptides can include a non-covalent interaction. The first and/or second polypeptide can contain a linker site. The complex can contain a linking molecule. The second polypeptide can contain at least two similar amino acid sequences at least five amino acids in length. The complex can contain an Fc-gamma receptor II blocking molecule (e.g., an anti-CD32 antibody).

Another aspect of the invention features a vaccine for vaccinating a mammal (e.g., human). The vaccine contains an Fc-gamma receptor II blocking molecule (e.g., an anti-CD32 antibody) and a polypeptide, where administration of the vaccine to the mammal induces an immune response against at least a portion of the polypeptide. The polypeptide can contain an amino acid sequence expressed by the mammal. The Fc-gamma receptor II blocking molecule and polypeptide can be connected, and the connection can include a non-covalent interaction.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

Other features and advantages of the invention will be apparent from the following detailed description, and from the claims.

### DESCRIPTION OF DRAWINGS

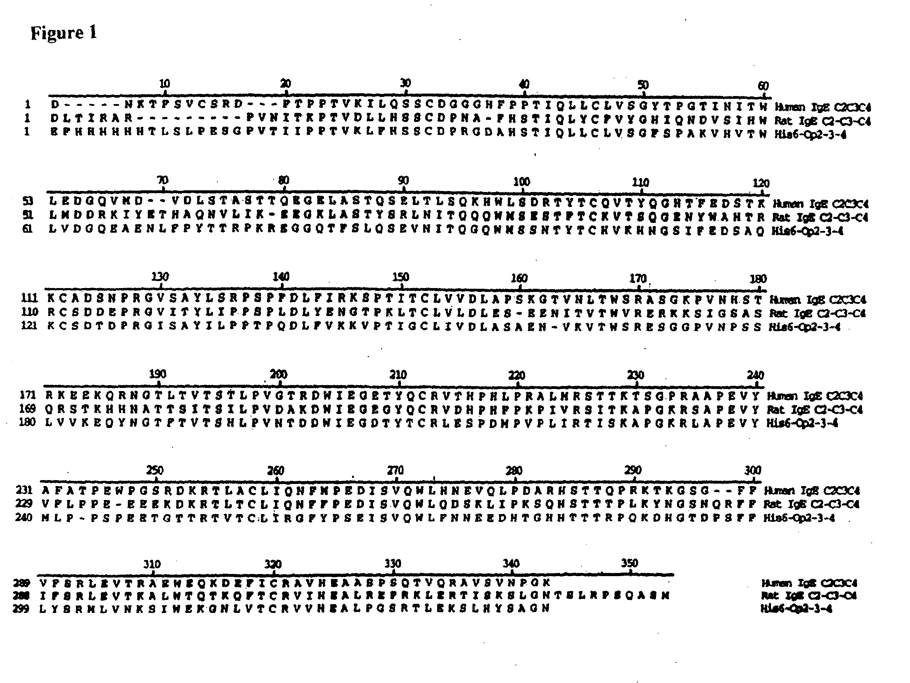

Figure 1 is a diagram comparing the amino acid sequences of the CH2-CH3-CH4 domains of human, rat, and opossum IgE, in the upper, middle, and lower rows, respectively. The opossum sequence also contains an N-terminal signal sequence followed by six histidine residues.

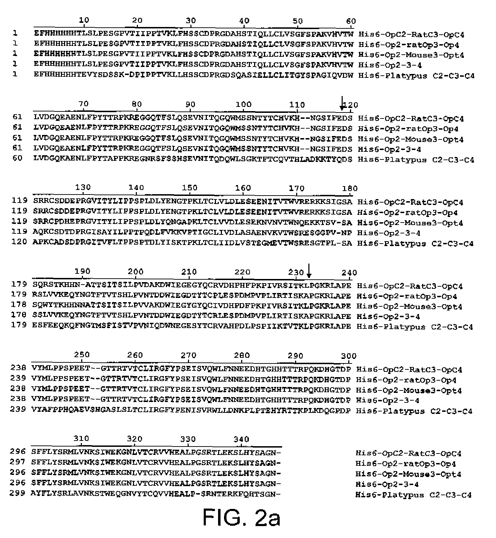

Figures 2A-B contain diagrams comparing the amino acid sequences of various polypeptides containing the following components: opossum CH2―rat CH3―opossum CH4 (ORO); opossum CH2―rat N-term CH3―opossum C-term CH3―opossum CH4 (ORO-trunc); opossum CH2―mouse CH3―opossum CH4 (OMO); opossum CH2―CH3―CH4 (OOO); platypus CH2―CH3―CH4 (PPP); opossum CH2―human CH3―opossum CH4 (OHO); opossum CH2―pig CH3―opossum CH4 (OPO); and opossum CH2―dog CH3―opossum CH4 (ODO). The arrows indicate domain borders.

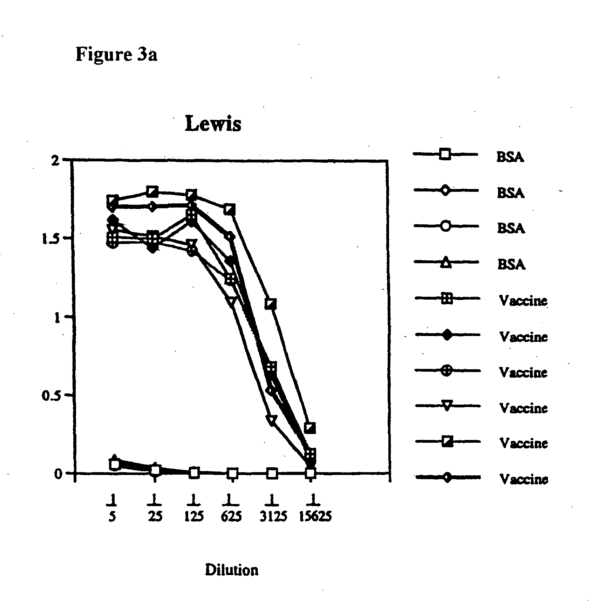

Figures 3A-C contain diagrams depicting the analysis of immune responses against an ORO immunogenic polypeptide in a panel of three different strains of rats. The level of rat IgG anti-IgE antibodies directed against native rat IgE was measured by an ELISA. Native rat IgE was used at a concentration of 5 µg/mL for coating of ELISA plates. Successive 1/5 dilutions of serum from each of the individual rats were tested by color reaction in the ELISA. Six vaccinated rats were analyzed together with four control rats from each strain.

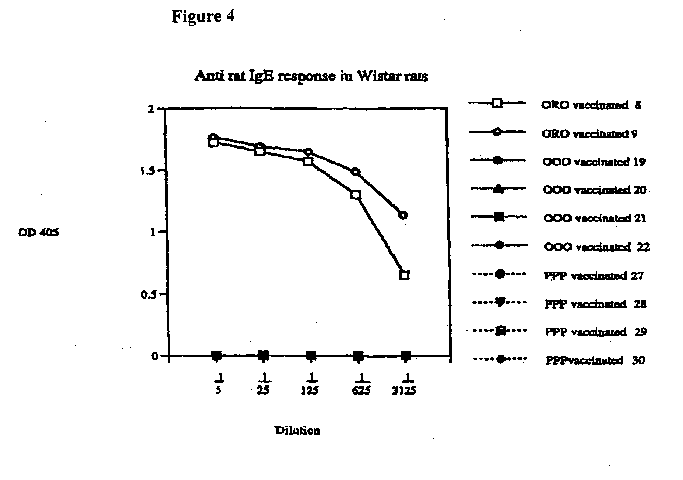

Figure 4 is a diagram depicting an analysis of the immune responses against an ORO immunogenic polypeptide as well as OOO and PPP control polypeptides.

### DETAILED DESCRIPTION

The invention provides methods and materials for the treatment of various diseases such as infections and IgE-related diseases. Specifically, the invention provides methods and materials that can be used to vaccinate a mammal against specific self or non-self antigens. For example, the methods and materials described herein can be used to reduce the effects of IgE antibodies within a mammal by reducing the amount of total and receptor bound IgE antibodies in the mammal.

### 1. Vaccine conjugates

The invention provides vaccine conjugates that contain at least two polypeptides with each of those polypeptides having at least two similar amino acid segments, The term "conjugate" as used herein refers to any composition containing at least two polypeptides that are directly or indirectly connected via one or more covalent or non-covalent bonds. For example, a conjugate can contain ten sequentially connected polypeptides (e.g., number one is connected to number two, number two is connected to number three, number three is connected to number four, etc.). The term "connected" as used herein with respect to polypeptides refers to any type of covalent or non-covalent bond including, without limitation, single bonds, double bonds, triple bonds, disulfide bonds, hydrogen bonds, hydrophobic interactions, van der Waals interactions, and any combination thereof. For example, a disulfide bond can connect polypeptide number one to polypeptide number two. Alternatively, an antibody can connect polypeptide numbers one and two. In this case, polypeptides one and two each would contain an epitope recognized by the antibody such that the resulting conjugate contains polypeptide number one non-covalently connected to the antibody which is non-covalently connected to polypeptide number two. It is noted that polypeptide numbers one and two in this example can have an identical amino acid sequence.

The term "amino acid segment" as used herein refers to a contiguous stretch of amino acid sequence within a polypeptide. For example, the amino acid sequence from residues 30 to 40 within a 100 amino acid polypeptide would be considered an amino acid segment. For the purpose of this invention, an amino acid segment can be any length greater than about five amino acid residues (e.g., greater than about six, seven, eight, nine, ten, 15, 20, 25, 30, 40, 50, 75, 100, 150, or 200 amino acid residues). Thus, an amino acid segment can be the entire CH3 domain of an IgE antibody.

The term "similar" as used herein with respect to at least two amino acid segments means the segments are at least about 50 percent identical in amino acid sequence. For example, similar amino acid segments can be about 50, 55, 60, 65, 70, 75, 80, 85, 90, 95, 99, or 100 percent identical. For the purpose of this invention, the percent amino acid sequence identity between one amino acid segment and another is calculated as follows. First, the amino acid sequences of the two amino acid segments are aligned using the MEGALIGN® (DNASTAR, Madison, WI, 1997) sequence alignment software following the Jotun Heim algorithm with the default settings. Second, the number of matched positions between the two aligned amino acid sequences is determined. A matched position refers to a position in which identical residues occur at the same position as aligned by the MEGALIGN® sequence alignment software. Third, the number of matched positions is divided by the total number of positions, and the resulting value multiplied by 100 to obtain the percent identity.

Again, a vaccine conjugate of the invention contains at least two polypeptides with each of those polypeptides having at least two similar amino acid segments. Thus, a vaccine conjugate can contain two, three, four, five, six, seven, eight, nine, ten, 15, 20, 25, or 30 polypeptides with each having at least two similar amino acid segments. It is noted that a polypeptide containing at least two similar amino acid segments can contain two, three, four, five, six, seven, eight, nine, ten, or more similar amino acid segments. In addition to the polypeptides containing at least two similar amino acid segments, a vaccine conjugate of the invention can contain any number of polypeptides not having at least two similar amino acid segments. For example, a vaccine conjugate can contain four polypeptides each having a 30 amino acid residue segment repeated three times as well as two polypeptides each lacking similar amino acid segments.

Typically, a vaccine conjugate contains a polypeptide that will act as an antigen against which an immune response is desired. Thus, a vaccine conjugate within the scope of the invention can contain any type of polypeptide including, without limitation, bacterial polypeptides, fungal polypeptides, viral polypeptides, and mammalian polypeptides. For example, a vaccine conjugate can contain five hepatitis C virus polypeptides. It is noted that each polypeptide of a conjugate can have an identical amino acid sequence. In addition, a polypeptide of a vaccine conjugate typically contains similar amino acid segments each of which can act as a defined antigenic unit against which an immune response is desired. Thus, a polypeptide of a vaccine conjugate can contain similar amino acid segments that correspond to any region from a polypeptide including, without limitation, receptor binding regions, ligand binding regions, enzyme active sites, enzyme cleavage sites of polypeptide substrates, antigen-binding regions of antibodies, and epitopes recognized by antibodies. For example, a polypeptide of a vaccine conjugate can contain three similar amino acid segments that each correspond to the enzyme active site of enzyme X. It is noted that similar amino acid segments can be in tandem or dispersed throughout a polypeptide. Typically, the administration of a vaccine conjugate results in the formation of antibodies having specificity for an epitope formed by at least a portion of the similar amino acid segments within one of the polypeptides of the vaccine conjugate.

Any method can be used to make the polypeptides of a vaccine conjugate including, without limitation, prokaryotic expression systems, eukaryotic expression systems, and chemical synthesis techniques. In addition, a polypeptide of a vaccine conjugate can be obtained from natural tissue sources. For example, a brain glycopolypeptide can be obtained from brain tissue. Typically, each different polypeptide of a conjugate is made independently, or isolated independently, and then used to form a conjugate. It is noted that polypeptides can be purified prior to being used to form a conjugate. Any method can be used to purify polypeptides including, without limitation, fractionation, centrifugation, and chromatography. For example, polypeptides containing a polyhistidine sequence can be purified using affinity chromatography. Once obtained, the polypeptides can be connected using any method. For example, a polypeptide sample can be incubated with a linking molecule such that individual polypeptides form conjugates. A linking molecule is any molecule that connects two polypeptides. Typically, a linking molecule is a molecule with two reactive groups or sites that are capable of interacting with and thereby forming a link between amino acid residues from two polypeptides. A linking molecule can be a specific linking molecule such as an antibody or a non-specific linking molecule such as a chemical reagent (e.g., glutaraldehyde and formaldehyde).

Any antibody can be used as a linking molecule. For example, an anti-polyhistidine antibody or an anti-epitope tag antibody such as an anti-FLAG® epitope antibody or anti-hemagglutinin (HA) tag antibody can be used to connect two polypeptides. FLAG® epitopes are described in U.S. Patent Numbers 4,703,004 and 4,782,137. It is noted that the polypeptides to be connected with a specific linking molecule need to contain the specific site recognized by the linking molecule. For example, to connect two polypeptides with an anti-polyhistidine antibody, each polypeptide must contain the polyhistidine epitope recognized by that antibody. For the purpose of this invention, the specific site recognized by a specific linking molecule such as an antibody is referred to as a linker site. Any method can be used to make a polypeptide that contains a linker site such that a particular antibody can be used as a linking molecule. For example, common molecular cloning techniques can be used to introduce the nucleic acid that encodes a FLAG tag epitope into the nucleic acid that encodes a particular polypeptide. It is noted that a linker site can be located at any position. For example, a polyhistidine sequence can be at the N-terminus, C-terminus, or an internal position of a polypeptide. In addition, a polypeptide can contain more than one linker site. For example, a polypeptide can have a polyhistidine sequence at an internal position as well as at the C-terminus. Further, a polypeptide can contain different linker sites. For example, a polypeptide can have a polyhistidine sequence at an internal position and a FLAG tag epitope at the C-terminus.

In some cases, two or more polypeptides can be made such that they are connected via a covalent bond. For example, two polypeptides can be made as a fusion protein such that they are connected by a peptide bond. Alternatively, a polypeptide can be made in a cell line that promotes the formation of disulfide bonds between, for example, two identical polypeptides. In this case, the conjugate would be a homodimer. It is noted that any polypeptide can be engineered to contain one or more cysteine residues such that the polypeptides form conjugates via cysteine bridges. For example, a polypeptide can be made to contain N- and C-terminal cysteine residues such that conjugates of varying size are formed intracellularly.

In addition, the interaction between biotin and avidin can be used to form conjugates. For example, polypeptides can be designed, or chemically treated, to contain biotin molecules at the C- and N-terminal ends. These biotin-containing polypeptide can be incubated with avidin molecules that are capable of simultaneously interacting with two or more biotin molecules. In this case, a single avidin molecule can link two biotin-containing polypeptides to form a conjugate. Further, chelating molecules that can simultaneously bind two or more ions (e.g., Ni⁺⁺, Cu⁺⁺, Co⁺⁺, and Zn⁺⁺) can be used to form conjugates. For example, a copper chelating molecule that can interact with two copper ions can be used to link two polypeptides containing a polyhistidine sequence. In this case, a single copper ion can interact with each polyhistidine sequence while a single copper chelating molecule links the two polypeptides to form a conjugate. It is noted that immunostimulating complexes (iscoms) can be used to form conjugates. For example, an iscom can be designed to contain copper ions such that polypeptides containing a polyhistidine sequence can be conjugated.

Typically, a nucleic acid molecule is constructed such that a particular polypeptide is expressed. For example, a nucleic acid molecule can be constructed to encode a polypeptide having three similar amino acid segments as well as a polyhistidine sequence at its C-terminus. Once constructed, the nucleic acid molecule can be introduced into a host cell such that the polypeptide is produced. Any host cell can be used including, without limitation, prokaryotic cells (e.g., bacteria) and eukaryotic cells (e.g., human cells). Once produced, the polypeptide can be purified and used to make the desired vaccine conjugate.

The term "nucleic acid" as used herein encompasses both RNA and DNA, including cDNA, genomic DNA, and synthetic (e.g., chemically synthesized) DNA. The nucleic acid can be double-stranded or single-stranded. Where single-stranded, the nucleic acid can be the sense strand or the antisense strand. In addition, nucleic acid can be circular or linear.

Nucleic acid can be obtained using common molecular cloning or chemical nucleic acid synthesis procedures and techniques, including PCR. PCR refers to a procedure or technique in which target nucleic acid is amplified in a manner similar to that described in U.S. Patent No. 4,683,195, and subsequent modifications of the procedure described therein. Generally, sequence information from the ends of the region of interest or beyond are used to design oligonucleotide primers that are identical or similar in sequence to opposite strands of a potential template to be amplified. Using PCR, a nucleic acid sequence can be amplified from RNA or DNA. For example, a nucleic acid sequence can be isolated by PCR amplification from total cellular RNA, total genomic DNA, and cDNA as well as from bacteriophage sequences, plasmid sequences, viral sequences, and the like. When using RNA as a source of template, reverse transcriptase can be used to synthesize complimentary DNA strands.

Any method can be used to introduce nucleic acid into a cell. In fact, many methods for introducing nucleic acid into cells, whether *in vivo* or *in vitro,* are well known to those skilled in the art. For example, calcium phosphate precipitation, electroporation, heat shock, lipofection, microinjection, and viral-mediated nucleic acid transfer are common methods for introducing nucleic acid into cells. In addition, naked DNA can be delivered directly to cells *in vivo* as describe elsewhere (U.S. Patent Number 5,580,859 and U.S. Patent Number 5,589,466 including continuations thereof). Further, nucleic acid can be introduced into cells by generating transgenic animals. It is noted that transgenic animals such as rabbits, goats, sheep, and cows can be engineered such that large amounts of a polypeptide are secreted into their milk.

Transgenic animals can be aquatic animals (such as fish, sharks, dolphin, and the like), farm animals (such as pigs, goats, sheep, cows, horses, rabbits, and the like), rodents (such as rats. guinea pigs. and mice), non-human primates (such as baboon, monkeys, and chimpanzees), and domestic animals (such as dogs and cats). Several techniques known in the art can be used to introduce nucleic acid into animals to produce the founder lines of transgenic animals. Such techniques include, but are not limited to, pronuclear microinjection (U.S. Patent No. 4,873,191); retrovirus mediated gene transfer into germ lines (Van der Putten *et al., Proc. Natl. Acad. Sci., USA*, 82:6148-6152 (1985)); gene transfection into embryonic stem cells (Gossler A *et al*., *Proc Natl Acad Sci USA* 83:9065-9069 (1986)); gene targeting into embryonic stem cells (Thompson *et al., Cell,* 56:313-321 (1989)); nuclear transfer of somatic nuclei (Schnieke AE *et al., Science* 278:2130-2133 (1997)); and electroporation of embryos.

For a review of techniques that can be used to generate and assess transgenic animals, skilled artisans can consult Gordon (*Intl. Rev. Cytol*., 115:171-229 (1989)), and may obtain additional guidance from, for example: Hogan *et al*., "Manipulating the Mouse Embryo" Cold Spring Harbor Press, Cold Spring Harbor, NY (1986); Krimpenfort *et al., Bio*/*Technology,* 9:844-847 (1991); Palmiter *et al., Cell, 41:343-*345 (1985); Kraemer *et al*., "Genetic Manipulation of the Early Mammalian Embryo" Cold Spring Harbor Press, Cold Spring Harbor, NY (1985); Hammer *et al., Nature,* 315:680-683 (1985); Purscel *et al., Science,* 244:1281-1288 (1986); Wagner *et al*., U.S. Patent No. 5,175,385; and Krimpenfort *et al*., U.S. Patent No. 5,175,384.

In addition, a nucleic acid that encodes a polypeptide can be maintained within a cell in any form. For example, nucleic acid can be integrated into the genome of a cell or maintained in an episomal state. In other words, a cell can be a stable or transient transformant.

Further, any method can be used to direct the expression of a particular polypeptide. Such methods include, without limitation, constructing a nucleic acid such that a regulatory element promotes the expression of a nucleic acid sequence that encodes a polypeptide. Typically, regulatory elements are DNA sequences that regulate the expression of other DNA sequences at the level of transcription. Thus, regulatory elements include, without limitation, promoters, enhancers, and the like.

In one embodiment, a conjugate to vaccinate rats can be designed to contain polypeptides having an N-terminal polyhistidine sequence followed by an opossum IgE CH2 domain, a rat IgE CH3 domain, an opossum IgE CH2 domain, a rat IgE CH3 domain, an opossum IgE CH4 domain, and a C-terminal polyhistidine sequence. Alternatively, the first opossum IgE CH2 domain can be followed by three rat IgE CH3 domains as opposed to only one rat IgE CH3 domain. In either case, two polypeptides can be connected via disulfide bonds such that dimers are formed. It is noted that affinity chromatography can be used to purify polypeptides containing a polyhistidine sequence. In addition, an anti-polyhistidine antibody can be used as a linking molecule to connect any number of single polypeptides or dimers through the N-terminal and C-terminal polyhistidine sequences. For example, three dimers can be linked sequentially via two anti-polyhistidine antibodies (i.e., dimer one connected to dimer two by antibody one, and dimer two connected to dimer three by antibody two). It is noted that mixing polypeptides with a linking molecule can result in a vaccine that contains vaccine conjugates with various sizes as well as various combinations of polypeptides. For example, a vaccine can contain a substantial amount of vaccine conjugates having less than four polypeptides with few having greater than four polypeptides. It is also noted that the general configuration of the polypeptides within a vaccine conjugate can be adapted to vaccinate mammals other than rats. For example, the rat IgE domains can be replaced with human IgE domains to vaccinate humans.

### 2. Vaccine conjugates and cytokines

The invention provides vaccine conjugates that contain a polypeptide having a cytokine activity such that a potent immune response is induced against another polypeptide within the conjugate. Such immune responses can be more potent than the responses induced by a conjugate lacking a polypeptide having a cytokine activity. Although not limited to any particular mode of action, a vaccine conjugate containing polypeptide X and a polypeptide having a cytokine activity presumably concentrates cytokine activity to the localized area containing polypeptide X. Thus, a vaccine conjugate containing a polypeptide having cytokine activity can stimulate cells that participate in generating a specific immune response against other polypeptides within a vaccine conjugate.

A polypeptide having cytokine activity can have any type of cytokine activity. For example, a polypeptide can have interferon-α, interferon-β, interferon-γ, TNF-α, IL-1, IL-2, IL-4, IL-6, IL-12, IL-15, IL-18, or granulocyte-macrophage colony stimulating factor (GM-CSF) activity. It is important to note that a polypeptide having cytokine activity can be a polypeptide that is either naturally occurring or non-naturally occurring. A naturally occurring polypeptide is any polypeptide having an amino acid sequence as found in nature, including wild-type and polymorphic polypeptides. Such naturally occurring polypeptides can be obtained from any species including, without limitation, human, chimpanzee, baboon, rat, or mouse. For example, human interferon-α can be used in a vaccine conjugate. A non-naturally occurring polypeptide is any polypeptide having an amino acid sequence that is not found in nature. Thus, a non-naturally occurring polypeptide can be a mutated version of a naturally occurring polypeptide, or an engineered polypeptide. For example, a non-naturally occurring polypeptide having interferon-α activity can be a mutated version of a naturally occurring polypeptide having interferon-α activity that retains at least some interferon-α activity. A polypeptide can be mutated by, for example, sequence additions, deletions, and/or substitutions using standard methods such as site-directed mutagenesis of the corresponding nucleic acid coding sequence.

A conjugate can contain any number of polypeptides having cytokine activity. For example, a conjugate can contain two polypeptides having cytokine activity. In addition, a conjugate can contain polypeptides having different cytokine activities. For example, a conjugate can contain one polypeptide having interferon-α activity and another having GM-CSF activity. It is noted that polypeptides having cytokine activity can be obtained using any method. For example, a polypeptide having cytokine activity can be designed to contain a polyhistidine sequence such that affinity chromatography can be used to purify the polypeptide. In addition, any method can be used to form a conjugate. For example, a polypeptide having cytokine activity can be designed to contain a linker site such that a linking molecule can link that polypeptide to another polypeptide such as any of the polypeptides described herein.

In one embodiment, a conjugate to vaccinate rats can be designed to contain polypeptides having cytokine activity as well as polypeptides having an N-terminal polyhistidine sequence followed by an opossum IgE CH2 domain, a rat IgE CH3 domain, an opossum IgE CH2 domain, a rat IgE CH3 domain, an opossum IgE CH4 domain, and a C-terminal polyhistidine sequence. In this case, the polypeptides having cytokine activity can contain an N-terminal polyhistidine sequence such that affinity chromatography can be used for purification. In addition, an anti-polyhistidine antibody can be used as a linking molecule to connect any number of polypeptides via the polyhistidine sequences. For example, a conjugate can contain an interferon-α polypeptide followed by three polypeptides containing IgE domains followed be an interferon-β polypeptide with each connection being via an anti-polyhistidine antibody. It is noted that mixing polypeptides with a linking molecule can result in a vaccine that contains vaccine conjugates with various sizes and various combinations of polypeptides. For example, a vaccine can contain a substantial amount of vaccine conjugates having polypeptides with interferon-α activity with few having both polypeptides with interferon-α activity and polypeptides with interferon-β activity. It is also noted that the general configuration of the polypeptides within a vaccine conjugate can be adapted to vaccinate mammals other than rats. For example, the rat IgE domains can be replaced with human IgE domains to vaccinate humans.

### 3. Immunogenic polypeptides and IgE vaccines

For a successful IgE vaccination, it is essential to obtain a strong immune response that reacts predominantly with native IgE molecules (e.g., IgE surface epitopes). This is required in order to achieve efficient competition with the IgE receptor for free IgE, as the interaction between an IgE antibody and its specific IgE receptor is very strong (2.6x10⁻¹⁰; Froese A, *CRC Crit. Rev. Immunol*. 1:79-132 (1980)). As described herein, high levels of antibodies having specificity for self IgE antibodies were produced in rat strains by administering an immunogenic polypeptide. Several different rat strains were used including low, medium, and high IgE responders.

An immunogenic polypeptide, as described herein, is a polypeptide that effectively induces an immune response in a mammal. For example, an immunogenic polypeptide can be a polypeptide that effectively induces an anti-self IgE response in a mammal. Typically, immunogenic polypeptides contain at least one amino acid sequence (e.g., a single amino acid substitution) that would be considered non-self to a particular mammal. For example, immunogenic polypeptides that induce anti-self IgE responses can contain two components: a self IgE portion and a non-self IgE portion. The self IgE portion can be responsible for conferring the specificity of the anti-self IgE response and the non-self IgE portion can serve to promote and stabilize the immunogenic polypeptide such that the specific anti-self IgE response is induced. Typically, the self IgE portion of the immunogenic polypeptide is a portion of an IgE antibody that either directly interacts with an IgE receptor or indirectly influences the interaction of an IgE antibody with an IgE receptor.

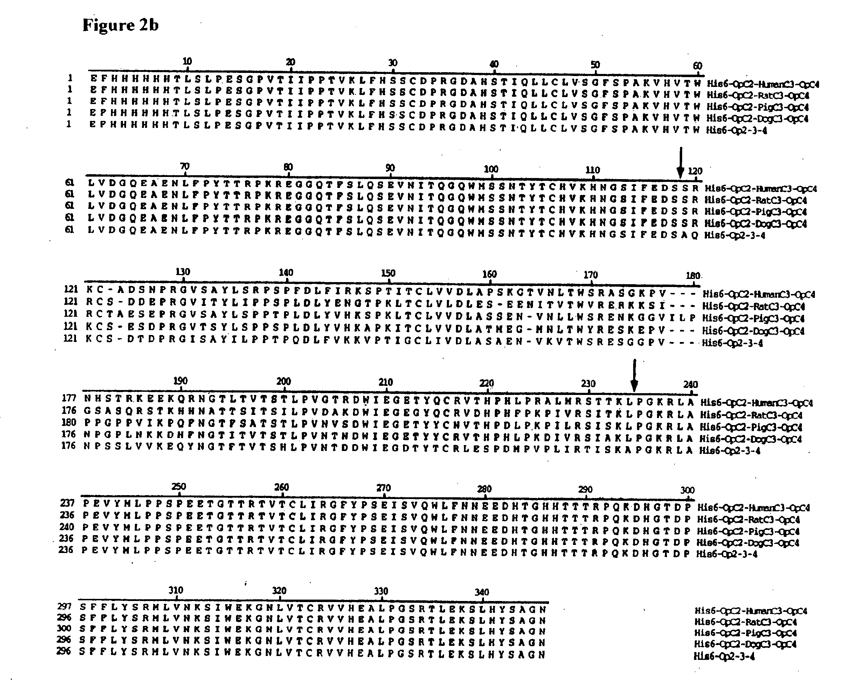

Briefly, the binding site for human IgE to the high affinity IgE receptor on mast cells and basophils is not located at the junction between the CH2 and CH3 domains of IgE as previously suggested, but instead is located in the N-terminal region of the CH3 domain. This region is, due to folding, located in the junction between the CH3 and CH4 domains of the native polypeptide. Thus, use of the entire CH2-CH3 domain as a self IgE portion may potentially induce an anti-self IgE response with antibodies interacting with self IgE antibodies already bound to the surface of mast cells such that anaphylactic reactions occur. To reduce the risk of inducing an anaphylactic response, the self IgE portion of an immunogenic polypeptide can be the entire CH3 domain without the CH2 domain. Alternatively, the self IgE portion can be the N-terminal region of the CH3 domain. For example, when vaccinating a rat, the self IgE portion can be the N-terminal half of the rat CH3 domain in a context of a non-self IgE portion containing the entire CH2 domain of opossum IgE, the C-terminal half of the CH3 domain of opossum IgE, and the entire CH4 domain of opossum IgE. Such an immunogenic polypeptide can be designated ORO-trunc (Figure 2).

Typically, the non-self IgE portion of an immunogenic polypeptide stabilizes a functional conformation of the self IgE portion. For example, if the self IgE portion is a CH3 domain, then the non-self IgE portion could be a CH2 domain, a CH4 domain, or a CH2 and CH4 domain with the self CH3 domain being between the CH2 and CH4 domains. Specifically, when vaccinating a rat, the self IgE portion can be the rat CH3 domain in a context of a non-self IgE portion from, for example, opossum. In this case, the rat CH3 domain can be located between the opossum CH2 and CH4 domains. Such an immunogenic polypeptide can be designated ORO (Figure 2). Likewise, when vaccinating a mouse, the self IgE portion can be the mouse CH3 domain in a context of a non-self IgE portion from, for example, opossum. Such an immunogenic polypeptide can be designated OMO (Figure 2).

Immunogenic polypeptides of the invention can be produced using a eukaryotic expression system, such as a mammalian cell expression system. In such cases, the immunogenic polypeptide is soluble, properly folded, and properly modified such that an anti-self IgE response is induced upon administration to a mammal. For example, immunogenic polypeptides having one or more eukaryotic post-translational modifications can produce an anti-self IgE response that is significantly higher than similar polypeptides lacking eukaryotic post-translational modification (e.g., a bacterially produced polypeptide). Eukaryotic post-translational modifications include, without limitation, glycosylation, acylation, limited proteolysis, phosphorylation, and isoprenylation. Further, soluble, properly folded, and properly modified immunogenic polypeptides can induce a strong anti-self IgE response in mammals with high concentrations of plasma IgE. so called high IgE responders. Bacterially produced polypeptides, however, are unable to produce such a strong anti-self IgE response in high IgE responders. Thus, immunogenic polypeptides having high solubility, proper folding, and proper modification can be obtained and used as described herein to induce effective anti-self IgE responses in mammals. Moreover, the immunogenic polypeptides described herein can be used to treat mammals, including humans, that have high serum concentrations of IgE. It is noted that a high percentage of the severely allergic patients in the human population belong to this category of patients.

The IgE CH3 domain, or a portion of an IgE CH3 domain, derived from an organism to be vaccinated such as human can be inserted into the structural context of a distantly related IgE molecule such as an IgE molecule from a non-placental mammal (e.g., opossum, platypus, koala, kangaroo, wallaby, and wombat). IgE antibodies from the grey short tailed opossum, a marsupial, exhibit about 25 percent sequence identity with human, rat, pig, and dog IgE antibodies. Thus, regions of the opossum IgE antibody can be used as the non-self IgE portion of an immunogenic polypeptide such that a potent anti-self IgE response is induced in a human, rat, pig, or dog.

A nucleic acid molecule for expressing an immunogenic polypeptide can be produced by splicing a first nucleic acid that encodes a portion of an IgE antibody from an organism to be vaccinated into a second nucleic acid that encodes a portion of an IgE antibody from a mammal distantly related to the organism to be vaccinated. For example, a nucleic acid molecule encoding an immunogenic polypeptide containing the CH3 domain of rat, human, pig, or dog IgE can be spliced into a nucleic acid containing the CH2 and CH4 domains of opossum IgE. Such chimeric nucleic acid molecules can be constructed using common molecular cloning techniques. In general, constructing nucleic acid such that the CH3 domain of an IgE antibody from one organism is positioned between the CH2 and CH4 domains of an IgE antibody from another organism results in a nucleic acid molecule that encodes a chimeric IgE molecule that has the CH3 domain in a structural context very similar to its native position within native IgE antibodies.

When vaccinating rat, human, dog, or pig, the opossum CH2 and CH4 domains can serve as the non-self IgE portion of the immunogenic polypeptide, since there is about 30 percent amino acid identity between opossum CH2 and CH4 domains and the corresponding domains of rat, human, dog, and pig IgE (Figure 1). Such immunogenic polypeptides can be produced in a mammalian host. In addition, the resulting immunogenic polypeptides can be secreted from the mammalian producer cells in a properly folded and properly glycosylated form. For example, analysis, in the Biacore system, with monoclonal antibodies directed against the CH3 domain of human IgE revealed that these monoclonal antibodies can bind strongly to immunogenic polypeptides of the invention, indicating that the entire CH3 domain can be properly folded.

It is important to note that immunogenic polypeptides described herein can be such that deleterious side-effects are not exhibited, even in mammals that have highly elevated IgE titres prior to vaccination. In addition, vaccination with an immunogenic polypeptide as described herein can induce an anti-self IgE response that is directed against the entire free pool of IgE. Such a response is not limited to a specific allergen. Thus, these methods and materials can be used to treat human allergies having a large variety of different atopic allergies.

### 4. Additional components and modes of administration

The vaccines, vaccine conjugates, and immunogenic polypeptides described herein can be administered alone or in combination with other components. For example, a vaccine conjugate can contain a blocking molecule that inhibits the interaction between an antibody (e.g., an IgG antibody) and an Fc-gamma receptor II (e.g., CD32). Such blocking molecules (i.e., Fc-gamma receptor II blocking molecules) can include, without limitation, anti-CD32 antibodies. Anti-CD32 antibodies can be obtained using common antibody production and screening techniques. It is noted that Fc-gamma receptor II blocking molecules can be used in combination with any immunogenic polypeptide such that the immune response against the immunogenic polypeptide is enhanced. For example, a mixture containing an anti-CD32 antibody and an immunogenic polypeptide either conjugated or not can be administered to a mammal to induce a potent immune response against the immunogenic polypeptide.

To vaccinate a mammal, an effective amount of any vaccine, vaccine conjugate, or immunogenic polypeptide described herein can be administered to a host. An effective amount refers to any amount that induces a desired immune response while not inducing significant toxicity to the host. Such an amount can be determined by assessing a host's immune response after administration of a fixed amount of a particular material (e.g., immunization polypeptide). In addition, the level of toxicity, if any, can be determined by assessing a host's clinical symptoms before and after administering a fixed amount of a particular material. It is noted that the effective amount of a particular material administered to a host can be adjusted according to desired outcomes as well as the host's response and level of toxicity. Significant toxicity can vary for each particular host and depends on multiple factors including, without limitation, the host's disease state, age, and tolerance to pain.

In addition, any of the materials described herein can be administered to any part of the host's body including, without limitation, the joints, blood stream, lungs, intestines, muscle tissues, skin, and peritoneal cavity. Thus, a vaccine conjugate can be administered by intravenous, intraperitoneal, intramuscular, subcutaneous, intrathecal, and intradermal injection, by oral administration, by inhalation, or by gradual perfusion over time. For example, an aerosol preparation containing an immunogenic polypeptide can be given to a host by inhalation. It is noted that the duration of vaccination with any of the materials described herein can be any length of time from as short as one day to as long as a lifetime (e.g., many years). For example, an immunogenic polypeptide can be administered once a year over a period of ten years. It is also noted that the frequency of treatment can be variable. For example, an immunogenic polypeptide can be administered once (or twice, three times, etc.) daily, weekly, monthly, or yearly.

Preparations for administration can include sterile aqueous or non-aqueous solutions, suspensions, and emulsions. Examples of non-aqueous solvents include, without limitation, propylene glycol, polyethylene glycol, vegetable oils, and injectable organic esters. Aqueous carriers include, without limitation, water as well as alcohol, saline, and buffered solutions. Preservatives, flavorings, and other additives such as, for example, antimicrobials, anti-oxidants, chelating agents, inert gases, and the like may also be present. It will be appreciated that any material described herein that is to be administered to a mammal can contain one or more commonly known pharmaceutically acceptable carriers.

Any method can be used to determine if a particular immune response is induced. For example, antibody responses against particular antigens can be determined using immunological assays (e.g., ELISA). In addition, clinical methods that can assess the degree of a particular disease state can be used to determine if a desired immune response is induced.

The invention will be further described in the following examples, which do not limit the scope of the invention described in the claims.

### EXAMPLES

### Example 1 - Immunogenic polypeptides

Nucleic acid molecules were constructed to encode immunogenic polypeptides containing both self and non-self IgE portions. These nucleic acid molecules were then used to synthesize soluble immunogenic polypeptides in mammalian cells. Such immunogenic polypeptides effectively induced a polyclonal anti-self IgE response upon administration to a mammal. In addition, the immunogenic polypeptides appear to be folded and glycosylated in a manner that enabled the immunogenic polypeptides to produce a strong and specific anti-self IgE response that was more potent than bacterially produced polypeptides lacking the non-self IgE portion. Thus, the immunogenic polypeptides described herein contain a majority of the surface epitopes in the same conformation as in native plasma IgE. Moreover, immunogenic polypeptides containing a self IgE portion limited to either the entire CH3 domain or a fragment of the CH3 domain (e.g., N-terminal region of CH3) reduced the potential of producing anaphylactic antibodies within a mammal.

### Example 2 - Production and purification of an immunogenic polypeptide

A ~330 base pair PCR fragment encoding the CH3 domain of rat IgE (Hellman L *et al., Nucleic Acids Res*. 10:6041-6049 (1982)) was fused with two similar sized fragments encoding the CH2 and CH4 domains of opossum IgE (Aveskogh M and Hellman L, *Eur. J Immunol*., 28:2738-2750 (1998)) by ligation into a modified version of the pCEP4 expression vector, pCEP-Pu2 (Margolskee RF *et al., Mol. Cell Biol.* 8:2837-2847 (1988)). This vector contains the CMV promoter-enhancer, located directly 5' of the coding region of interest and allows high levels of expression in mammalian cells. This vector also contains the coding regions for puromycin resistance and the EBV EBNA1 gene. The EBNA1 gene confers maintenance of stable replicating episomal copies of the vector in human or canine cell lines.

The nucleic acid molecule containing the opossum IgE CH2, rat IgE CH3, and opossum IgE CH4 nucleic acid sequences also contained nucleic acid sequences that encode a signal sequence and six histidine residues at the N-terminal region. The region containing the signal sequence and six histidine residues facilitates secretion of the encoded polypeptide from producer cells and enables polypeptide purification with Ni⁺⁺-chelating columns. Following transfection of the expression vector into human 293 cells, the opossum CH2-IgE/rat CH3-IgE/opossum CH4-IgE (ORO) immunogenic polypeptide was purified from 293 cell conditioned media on a nickel-chelating column to about 100 percent purity. Following elution of the ORO immunogenic polypeptide with a solution containing 20 mM Tris (pH 8.0), 0.1 M NaCl, and 100 mM imidazole, the eluate was dialyzed against PBS (pH 7.5) overnight at 4°C. The ORO immunogenic polypeptide was then concentrated to about 2 mg/mL using an Amicon concentrator. An aliquot of this preparation containing the ORO immunogenic polypeptide was separated on SDS-PAGE and found to be about 100 percent pure. This purified ORO immunogenic polypeptide preparation was used as the active component of an anti-self IgE vaccine for treating rats.

### Example 3 - Sensitization procedure

Each rat was sensitized to ovalbumin as follows. Ten (10) µg of ovalbumin in PBS was administered to each rat intraperitoneally. Three weeks after this initial intraperitoneal injection of ovalbumin, the rats received weekly intraperitoneal injections of 1 µg of ovalbumin for four weeks. During this four week period, the rats became sensitized to ovalbumin obtaining a total IgE and ovalbumin-specific IgE response that was high and persistent. After this four week period, the rats started a vaccination program. During the entire vaccination program, intraperitoneal injections of ovalbumin continued as follows. During the first two weeks of vaccination, the rats received intraperitoneal injections of 1 µg of ovalbumin weekly. After the first two weeks of vaccination, the rats received intraperitoneal injections of 1 µg of ovalbumin every other week.

### Example 4 - ELISA measurement of an anti-self IgE response

Thirty-six rats (twelve Lewis rats, twelve Louvain rats, and twelve Brown Norway rats) were divided into two equally sized groups and injected intraperitoneally with either the ORO immunogenic polypeptide or BSA as negative control. The BSA negative control was used at the same polypeptide concentration as that of the ORO immunogenic polypeptide. In this study, each rat received intraperitoneal injections of about 250 µg of antigen (either the ORO immunogenic polypeptide or BSA) dispersed in 0.2 mL of a 50:50 solution of Freund's complete adjuvant and PBS. Three weeks later, the rats were given a booster injection containing about 100 µg of antigen dispersed in 0.1 mL of a 50:50 solution of Freund's incomplete adjuvant and PBS. Six weeks later, the rats were given an additional booster identical to the first booster. One week after this third immunization, blood samples were taken and measured in an ELISA as follows.

The level of IgG anti-IgE antibodies directed against self rat IgE was measured by an ELISA. Native rat IgE was used at a concentration of 5 µg/mL for coating the ELISA plates. Successive 1/5 dilutions of serum from each of the individual rats were tested by color reaction in the ELISA. The presence of rat IgG antibodies having specificity for rat IgE antibodies was determined using two biotinylated mouse monoclonal antibodies, one with specificity for rat IgG2a/b and one for rat IgG1. Alkaline phosphatase coupled strepavidin was used to detect these biotinylated mouse monoclonal antibodies.

### Example 5 - Induction of an anti-self IgE response in a mammal

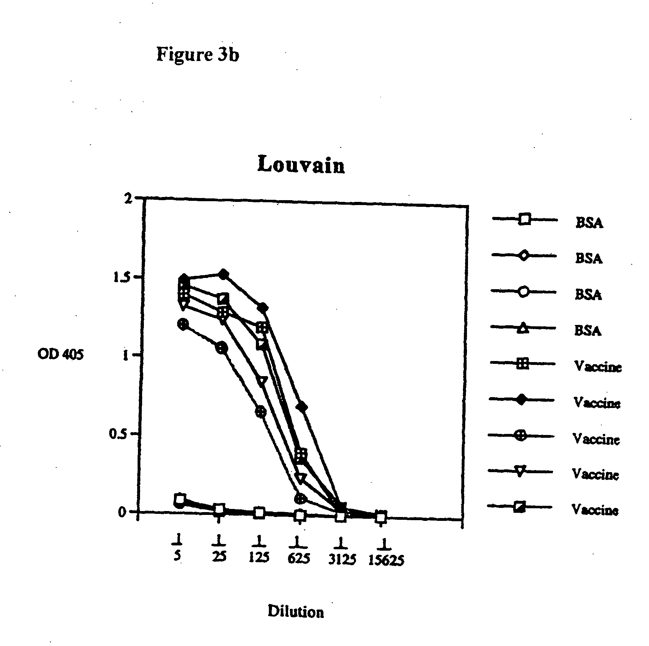

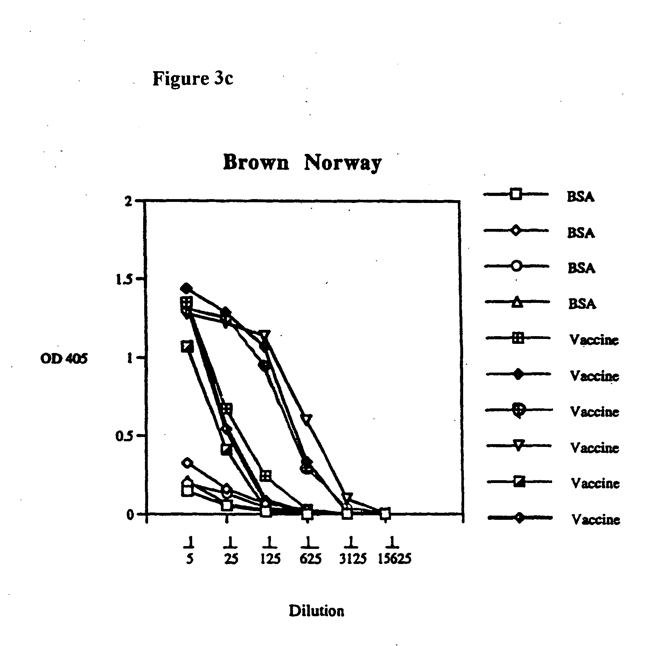

The *in vivo* effect of the ORO immunogenic polypeptide as an IgE vaccine was investigated using three different strains of rats (Lewis, Louvain, and Brown Norway). Lewis rats are low IgE responders, Louvain rats are medium IgE responders, and Brown Norway rats are high IgE responders. After sensitization to ovalbumin, each rat was vaccinated with either the ORO immunogenic polypeptide or BSA as described in Example 3. After collecting blood samples, the sera was diluted in steps of five as indicated (Figure 3). Purified monoclonal rat IgE (IR 162) was used to coat the ELISA plates (5 µg/mL) and two biotinylated mouse monoclonal antibodies were used to detect rat IgG anti-IgE antibodies. Following the second booster dose, high anti-IgE titres were detected in the low, medium, and high IgE responding rats that received the vaccine containing the ORO immunogenic polypeptide. Anti-IgE titers were not detected in rats treated with the BSA control. Thus, the ORO immunogenic polypeptide was capable of inducing an anti-self IgE response in rats that contained low, medium, and high amounts of IgE antibodies.

A difference in anti-self IgE levels between the various strains was observed. The low responder strain, Lewis, showed very high anti-self IgE titres. The sera could be diluted more than 3000 times before a significant decrease in OD values upon ELISA measurements was detected (Figure 3). For the high responder strain, Brown Norway, however, the OD values started to drop for three out of six animals at a dilution of 25 times or more (Figure 3).

In another experiment, Wistar rats were used. Wistar rats are medium IgE responders. The anti-self IgE response produced by the Wistar rats was similar to the response observed in the Lewis rats.

### Example 6 - Analysis of cross reactivity

The cross reactivity between rat antibodies directed against an opossum IgE CH2 or CH4 domain with the corresponding domain of rat IgE antibodies was evaluated. This potential cross reactivity could result from a low primary amino acid sequence homology or a close structural similarity between the CH2 and CH4 domains of opossum IgE and rat IgE. The induction of an anti-rat IgE response having specificity for the rat CH2 or CH4 domains could lead to mast cell activation.

A recombinant polypeptide (OOO) containing the opossum CH2-CH3-CH4 domains was injected into the Wistar strain. After a second booster injection, sera from these rats were collected and tested for the presence of antibodies having specificity for rat IgE. No anti-rat IgE antibodies were detected in the rats treated with the OOO polypeptide (Figure 4). In addition, Wistar rats treated with the ORO immunogenic polypeptide exhibited an anti-self IgE response similar to that observed in Lewis rats. Further, Wistar rats treated with a recombinant polypeptide (PPP) containing platypus CH2-CH3-CH4 domains did not produce an anti-rat IgE response. Thus, the CH2, CH3, and CH4 domains of opossum and platypus IgE antibodies do not generate, upon administration to rats, rat antibodies having specificity for rat IgE antibodies.

The interaction between the rat antibodies induced by the ORO immunogenic polypeptide (rat IgG anti-self IgE antibodies) and human IgE antibodies was examined. In a few cases, minor cross reactivity was observed. This minor cross reactivity detected in a few rats was most likely caused by the interaction between rat IgG anti-rat CH3 IgE antibodies and the CH3 domain of human IgE. Since the CH3 domains of rat and human IgE are much more closely related than human and opossum or platypus IgE, vaccines containing opossum or platypus components can be considered highly safe, presenting minimal risk for the generation of cross linking antibodies.

### Example 7 - Polypeptides for vaccine conjugates

The nucleic acid construct encoding the ORO immunogenic polypeptide described in Example 2 was used to produce two polypeptides each having several identical self epitopes. One polypeptide contained two identical clusters of self epitopes (the entire rat CH3 domain), while the other polypeptide contained four such clusters. First, nucleic acid encoding six histidine residues was added to the C-terminal end of the opossum CH4 domain by including a nucleotide sequence for six histidine residues in the 3' PCR primer. Thus, each polypeptide contained a polyhistidine sequence at both the N- and C-terminal ends so that conjugates can be formed. Second, a nucleic acid fragment encoding the opossum CH2 and the rat CH3 domains of the original construct was obtained by PCR amplification. This fragment was subsequently ligated into the construct encoding the ORO immunogenic polypeptide. The resulting construct encoded a polypeptide designated ORORO. This ORORO polypeptide contains two rat CH3 domains, two opossum CH2 domains, and one opossum CH4 domain in the following order opossum CH2, rat CH3, opossum CH2, rat CH3, and opossum CH4. Thus, this polypeptide has two identical CH3 domains, each with multiple self epitopes.

The nucleic acid construct encoding ORORO was used as starting material to produce the second immunogenic polypeptide. This polypeptide contains two additional rat CH3 domains that were added to a position 3' of the first rat CH3 domain in the ORORO polypeptide. The resulting polypeptide has a polyhistidine tag followed by an opossum CH2 domain, three identical rat CH3 domains, one opossum CH2 domain, one rat CH3 domain, an opossum CH4 domain, and a C-terminal polyhistidine tag (6his-ORRRORO-6his).

Each recombinant polypeptide was produced in the pCEP4 based vector system. In addition, the polypeptides were purified using Ni⁺⁺ chelating columns according to the method described in Example 2. Similar vaccine constructs are produced using dog or human IgE CH3 domain instead of the rat IgE CH3 domain.

### Example 8 - Vaccine conjugates

To determine a favorable combination of polypeptide to monoclonal antibody, the purified polypeptides of Example 7 are mixed with a monoclonal anti-polyhistidine antibody in various combinations ranging from a 1/1 to a 10/1 ratio (polypeptide to monoclonal antibody ratio). This mixture results in the generation of long multimeric conjugates with a large number of identical self epitopes in tandem. The biological activity of the various combinations is assessed in rats as described herein. The non-conjugated ORO immunogenic polypeptide is used as a reference to assess immune responses.

### Example 9 - Polypeptides having cytokine activity

PCR primers are designed so that cDNAs encoding rat, dog, and human cytokines (e.g., interferon-α, interferon-γ, and GM-CSF) can be isolated from total spleen mRNA. The nucleotide sequence encoding six histidine residues is introduced into each 5' PCR primer so that the cytokines can be purified via affinity chromatography and can be non-covalently conjugated to the polypeptides described herein via an anti-polyhistidine antibody. The recombinant cytokines are produced using any one of the following three expression systems: bacteria, yeast (e.g., *Pichia pastoris*), and mammalian cells (e.g., 293-EBNA cells using a pCEP-4 based expression system).

### Example 10 - Vaccine conjugates having a polypeptide with cytokine activity

Cytokines produced according to Example 9 are used to make vaccine conjugates. A mixture of three different cytokines (e.g., mouse interferon-α, rat interferon-γ and rat GM-CSF) is produced and tested in combination with the ORORO polypeptide and an anti-polyhistidine antibody.

Initially, a mixture of 1/10 ratio (cytokine to immunogenic polypeptide ratio) is tested. With three cytokines, this ratio results in a mixture of 30% cytokine per molar basis and 70% immunogenic polypeptide. In addition, this mixture contains an anti-polyhistidine antibody at a 1/10 ratio (immunogenic polypeptide to monoclonal antibody ratio). A large number of ratio combinations is evaluated so that an optimal cytokine to immunogenic polypeptide ratio as well as an optimal monoclonal antibody to immunogenic polypeptide ratio is determined. In addition, polypeptides having cytokine activity from various species is assessed to determine the optimal combination for a particular species.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

The invention provides an immunogenic polypeptide, comprising a self IgE portion and non-self IgE portion, wherein said immunogenic polypeptide is effective to induce an anti-self IgE response in a mammal, in particular a human. The self portion can comprise at least a portion of a CH3 domain of IgE. This polypeptide can be capable of dimerizing to form soluble immunogenic dimer effective to induce said anti-self IgE response in a mammal. The non-self IgE portion can comprise a first region and the second region, and the self IgE portion can be located between the first and second regions of the non-self IgE portion. The first region of the polypeptide can comprise at least a portion of an IgE CH2 domain and the second region can comprise at least a portion of an IgE CH4 domain. The non-self IgE portion can comprise an IgE sequence present in a non-placental mammal. The non-placental mammal is selected from the group consisting of opossum, platypus, koala, kangaroo, wallaby and wombat. The self IgE portion can lack the CH2 domain of an IgE antibody. The immunogenic polypeptide can contain eukaryotic post-translational modification. The immunogenic polypeptide can comprise a polyhistidine sequence. The anti-self IgE response induced by the immunogenic polypeptide can be a polyclonal response.

The invention also provides a nucleic acid molecule comprising a nucleic acid sequence that encodes the immunogenic polypeptide, the immunogenic polypeptide comprising a self IgE portion and a non-self IgE portion, wherein said immunogenic polypeptide is effective to induce an anti-self IgE response in a mammal. The nucleic acid molecule can comprise an additional nucleic acid sequence that encodes an amino acid sequence that promotes the secretion of the immunogenic polypeptide from a eukaryotic cell.

The invention also includes a host cell, comprising a nucleic acid molecule, wherein said nucleic acid molecule comprises a nucleic acid sequence that encodes an immunogenic polypeptide, said immunogenic polypeptide comprising a self IgE portion and a non-self IgE portion, wherein said immunogenic polypeptide is effective to induce an anti-self IgE in response in a mammal. The host cell can be a eukaryotic cell.

The invention also includes a soluble immunogenic dimer, comprising two immunogenic polypeptides that are capable of dimerizing to form the soluble immunogenic dimer, wherein each of the two immunogenic polypeptides comprises a self IgE portion and a non-self IgE portion, the soluble immunogenic dimer being effective to induce an anti-self IgE response in a mammal.

The invention also includes a vaccine, comprising an immunogenic polypeptide, the immunogenic polypeptide comprising a self IgE portion and a non-self IgE portion, wherein the immunogenic polypeptide is effective to induce an anti-self IgE response in a mammal. The vaccine can further comprise pharmaceutically acceptable carrier.

The invention also includes a method for making a nucleic acid molecule that encodes an immunogenic polypeptide effective to induce an anti-self IgE response in a mammal, the method comprising combining first and second nucleic acid sequences to form the nucleic acid molecule, wherein the first nucleic acid sequence encodes at least a portion of an IgE molecule present within the mammal, and wherein the second nucleic acid sequence encodes at least a portion of an IgE molecule not present in the mammal.

The invention also includes a method for making a nucleic acid molecule that encodes an immunogenic polypeptide effective to induce an anti-self IgE response in a mammal, the method comprising a) selecting a first nucleic acid sequence, wherein the first nucleic acid sequence encodes at least a portion of an IgE molecule present within the mammal b) selecting a second nucleic acid sequence, wherein the second nucleic acid sequence encodes at least a portion of an IgE molecule not present in the mammal, and c) combining the first and second nucleic acid sequences to form the nucleic acid molecule.

The invention also includes a vaccine complex for vaccinating a mammal, the complex comprising a first and second polypeptide, wherein each of the first and second polypeptide contains at least two similar amino acid sequences at least five amino acid residues in length, wherein the first and second polypeptides are connected to form the complex, and wherein administration of the complex to the mammal induces an immune response against at least a portion of the first or second polypeptide. The mammal can be a human. The first or second polypeptide can comprise an amino acid sequence expressed by the mammal. The first and second polypeptides can be identical. The first and second polypeptides can form a dimer. The connection of the first and second polypeptides can comprise a disulphide bond. The connection of the first and second polypeptides can comprise a non covalent interaction. The first or second polypeptide can comprise a linker site. The linker site can be a polyhistidine sequence. The amino and carboxyl termini of the first and second polypeptide can contain a linker site. The complex can comprise a linking molecule. The linking molecule can connect the first and second polypeptide. The linking molecule can comprise an antibody. The antibody can be anti polyhistidine antibody. The complex can comprise a third polypeptide, the third polypeptide having a cytokine activity. The cytokine activity can be an activity of a cytokine selected from the group consisting of interferon-α, interferon-β , interferon-γ, TNF-α , IL-1, IL-2, IL-4, IL-6, IL-12, IL-15, IL-18, and granulocyte-macrophage colony stimulating factor. The linking molecule can connect the third polypeptide to the first and second polypeptide. The first and second polypeptides can comprise a linker site. The amino and carboxyl termini of the first and second polypeptide can contain a linker site. The similar amino acid sequences of the complex can be greater than in about twenty amino acid residues in length. The complex can comprise an Fc-gamma receptor II blocking molecule.

The invention also includes a vaccine complex for vaccinating a mammal, the complex comprising a first polypeptide connected to a second polypeptide, wherein the first polypeptide contains at least two similar amino acid sequence at least five amino acids in length, wherein the second polypeptide has a cytokine activity, and wherein administration of the complex to the mammal induces an immune response against at least a portion of the first polypeptide. The mammal can be a human. The first polypeptide can comprise an amino acid sequence expressed by the mammal. The connection of the first and second polypeptide can comprise a non-covalent interaction. The first or second polypeptide can comprise a linker site. The complex can comprise a linking molecule. The cytokine activity can be an activity of a cited cytokine selected from the group consisting of interferon-α, interferon-β , interferon-γ, TNF-α , IL-1, IL-2, IL-4, IL-6, IL-12, IL-15, IL-18, and granulocyte-macrophage colony stimulating factor. The complex can comprise a third polypeptide. The first and third polypeptide can be identical. The first and third polypeptide can form a dimer. The similar amino acid sequences can be greater than about twenty amino acid residues in length. The complex can comprise an Fc-gamma receptor II blocking molecule.

The invention also includes a vaccine complex for vaccinating a mammal, the complex comprising a first, second and third polypeptide, wherein the first, second and third polypeptides are connected to form the complex, wherein the first polypeptide has a first cytokine activity, wherein the second polypeptide has a second cytokine activity, and wherein administration of the complex to the mammal induces an immune response against at least a portion of the third polypeptide. The mammal can be a human. The third polypeptide can comprise an amino acid sequence expressed by the mammal. The connections of the first, second and third polypeptides can comprise non-covalent interactions. The first, second or third polypeptide can comprise a linker site. The complex can comprise a linking molecule. The third polypeptide can comprise at least two similar amino acid sequences at least five amino acids in length. The complex can comprise a Fc-gamma receptor II blocking molecule.

The invention also includes a vaccine complex for vaccinating a mammal, the complex comprising a first polypeptide connected to a second polypeptide, wherein the first polypeptide is a polypeptide having interferon-α or interferon-β, and wherein administration of the complex to the mammal induces an immune response against at least a portion of the second polypeptide. The mammal can be a human. The second polypeptide can comprise an amino acid sequence expressed by the mammal. The connection of the first and second polypeptide can comprise a non-covalent interaction. The first or second polypeptide can comprise a linker site. The complex can comprise a linking molecule. The second polypeptide can comprise at least two similar amino acid sequences at least five amino acids in length. The complex can comprise a Fc-gamma receptor II blocking molecules.

## Claims

1. A polypeptide comprising a first amino acid segment greater than 20 residues in length present in a human IgE CH3 domain and a second amino acid segment greater than 20 residues in length present in an IgE domain of a non-placental mammal.

2. The polypeptide of claim 1, wherein said second amino acid segment is present in an IgE CH2 or CH4 domain of a non-placental mammal.

3. The polypeptide of claim 1, wherein said polypeptide is capable of dimerizing to form a soluble dimer.

4. The polypeptide of claim 1, wherein said second amino acid segment comprises a first region and a second region, and wherein said first amino acid segment is located between said first and second regions.

5. The polypeptide of claim 4, wherein said first region comprises at least a portion of an IgE CH2 domain.

6. The polypeptide of claim 4, wherein said second region comprises at least a portion of an IgE CH4 domain.

7. The polypeptide of claim 4, wherein said first region of said amino acid segment consists essentially of a CH2 domain of IgE, and wherein said second region of said second amino acid segment consists essentially of a CH4 domain of IgE.

8. The polypeptide of claim 1, wherein said non-placental mammal is selected from the group consisting of opossum, platypus, koala, kangaroo, wallaby, and wombat.

9. The polypeptide of claim 1, wherein said polypeptide comprises a human IgE CH3 domain located between an opossum IgE CH2 domain and an opossum IgE CH4 domain.

10. The immunogenic polypeptide of claim 1, wherein said immunogenic polypeptide comprises a polyhistidine sequence.
